Europäisches Patentamt

European Patent Office

Office européen des brevets

(11) Numéro de publication : **0 386 078 B1**

# (12) FASCICULE DE BREVET EUROPEEN

(45) Date de publication du fascicule du brevet :
**19.08.92 Bulletin 92/34**

(21) Numéro de dépôt : **88909766.3**

(22) Date de dépôt : **12.11.88**

(86) Numéro de dépôt international :
**PCT/EP88/01027**

(87) Numéro de publication internationale :
**WO 89/04679 01.06.89 Gazette 89/12**

(51) Int. Cl.⁵ : **A61M 5/24,** A61M 5/28,
A61M 5/20

(54) **APPAREIL DISTRIBUTEUR DE SUBSTANCES LIQUIDES.**

(30) Priorité : **16.11.87 CH 4448/87**

(43) Date de publication de la demande :
**12.09.90 Bulletin 90/37**

(45) Mention de la délivrance du brevet :
**19.08.92 Bulletin 92/34**

(84) Etats contractants désignés :
**AT BE CH DE FR GB IT LI LU NL SE**

(56) Documents cités :
**EP-A- 0 219 899**
**WO-A-87/06141**

(73) Titulaire : **MEDICORP HOLDING S.A.**
**11 Boulevard du Prince Henri**
**L-2014 Luxembourg (LU)**

(72) Inventeur : **MEYER, Gabriel**
**10a, chemin des Princes**
**CH-1222 Vesenaz (CH)**
Inventeur : **HOWALD, Ernst**
**10b, chemin des Princes**
**CH-1222 Vesenaz (CH)**

(74) Mandataire : **Nithardt, Roland**
**Cabinet Roland Nithardt Conseils en Propriété**
**Industrielle S.A. Y-Parc Scientifique et**
**Technologique Chemin de la Sallaz**
**Casepostale 3347**
**CH-1400 Yverdon-les-Bains (CH)**

# Description

La présente invention concerne un appareil distributeur de substances liquides en particulier de produits médicamenteux, comportant un réservoir allongé ouvert à une de ses extrémités, comprenant un fond fermé à son extrémité opposée, des parois latérales sensiblement cylindriques et un col rétréci, et contenant au moins un liquide à mélanger à une autre substance ou à distribuer, par exemple par pulvérisation, par application cutanée ou oculaire, ou par injection; et un organe distributeur, mobile par rapport à ce réservoir, cet organe distributeur comportant au moins un organe obturateur, cet organe obturateur, ayant également une fonction de piston et de vanne, étant partiellement engagé dans le col rétréci du réservoir et étant agencé pour obturer ce réservoir lorsque l'appareil est dans une première position de stockage et pour libérer le liquide lorsque l'appareil est dors une deuxième position activée; ainsi qu'une capsule solidaire de cet organe obturateur et montée sur le réservoir de telle manière qu'un tronçon d'extrémité de ce réservoir, voisin de son col rétréci, soit engagé dans cette capsule; ce réservoir étant coiffé d'un capuchon équipé d'organes d'inviolabilité le rendant solidaire de la capsule pendant le stockage.

Il existe déjà des appareils distributeurs de ce type qui sont géréralement conçus pour être stockés pendant une période pouvant être relativement longue. Un tel appareil distributeur est décrit dans la publication WO-A-87/06141. L'organe obturateur comporte une tête en élastomère qui est fortement comprimée à l'intérieur du col rétréci du réservoir. Cet organe obturateur comporte par ailleurs des organes de retenue élastiques qui ont tendance à le retenir dans sa position et empêcher tout déplacement vers l'intérieur du réservoir. Enfin, il règne à l'intérieur de ce réservoir une légère surpression qui oppose également une force tendant à empêcher la pénétration accidentelle de l'organe obturateur à l'intérieur du réservoir pendant toute la période du stockage.

Il en résulte que pour amener l'organe obturateur de la position qu'il occupe pendant que l'appareil est dans sa position de stockage vers la position qu'il occupe lorsque l'appareil est dans sa position activée, il est nécessaire d'exercer une certaine force susceptible de vaincre les contraintes initiales. Si cette force est exercée sans contrôle ni retenue, une partie du liquide initialement contenu dans le réservoir risque d'être éjectée et perdue. Etant donné que le volume de ce liquide contenu dans le réservoir est dosé de façon précise, surtout lorsqu'il s'agit d'un médicament, par exemple d'un médicament à injecter, il est essentiel qu'une partie de ce liquide ne soit pas perdue au cours des manipulations décrites ci-dessus.

La présente invention se propose de résoudre ce problème en prévoyant en particulier des moyens permettant de contrôler de façon précise le passage de l'appareil de sa position de stockage à sa position activée.

Dans ce but, cet appareil est caractérisé en ce que le capuchon est pourvu d'un poussoir qui est agencé pour prendre appui sur au moins une partie du fond fermé du réservoir et/ou de ses parois latérales, et qui est conçu pour pousser le réservoir de telle manière que l'organe obturateur pénètre dans le col et que l'appareil passe de sa position de stockage à sa position activée, ce poussoir étant une pièce mobile axialement par rapport audit capuchon.

Selon un mode de réalisation particulièrement avantageux, l'organe distributeur est une aiguille de seringue et le liquide à distribuer est un médicament injectable.

Selon un autre mode de réalisation très avantageux, l'organe distributeur est un applicateur cutané, oculaire ou un pulvérisateur et le liquide est une substance à distribuer par voie cutanée, oculaire ou par pulvérisation.

Pour permettre de limiter la course du poussoir et de ce fait assurer un contrôle précis du déplacement du réservoir pour amener l'appareil de sa position de stockage à sa position activée, le capuchon comporte avantageusement au moins une butée d'arrêt.

Dans certaines utilisations, la quantité de liquide contenu dans le réservoir ne correspond plus à une dose unitaire mais à un volume qui peut correspondre à plusieurs multiples d'une dose unitaire. Dans ce cas, le capuchon comporte avantageusement deux butées d'arrêt agencées pour définir la course du poussoir pour chaque dose de liquide à distribuer et des moyens pour assurer un déplacement axial contrôlé du capuchon pour ramener le poussoir dans sa position initiale après chaque distribution d'une dose de liquide.

Ces moyens destinés à assurer le déplacement axial contrôlé du capuchon comportent de préférence un filetage intérieur du capuchon et un élément complémentaire agencé pour s'engager dans ce filetage qui est solidaire de la capsule.

Dans le cas où le réservoir comporte une dose unitaire d'un seul liquide à distribuer, la course du poussoir est de préférence égale au déplacement axial du réservoir, par rapport à l'organe d'obturation, nécessaire pour amener la première goutte du liquide à une extrémité de distribution de l'organe distributeur. Dans le cas d'une seringue, cette opération est couramment appelée "le débullage" de la seringue.

Dans le cas où le réservoir comporte deux compartiments séparés par un deuxième organe obturateur et où le compartiment disposé entre le premier organe obturateur et le deuxième organe obturateur, et correspondant au col rétréci du réservoir, contient un liquide destiné à être mélangé à une substance contenue dans le deuxième compartiment, lui-même délimité entre le deuxième organe obturateur et le fond du réservoir, la course du poussoir est de préférence égale au déplacement axial du réservoir, par rapport au premier organe d'obturation, nécessaire pour assurer l'éjection du deuxième organe d'obturation hors du col rétréci afin de permettre le passage du liquide du premier compartiment dans le deuxième compartiment.

Dans cet exemple de réalisation, le deuxième compartiment contient de préférence une substance à mélanger au liquide du premier compartiment.

Selon un mode de réalisation particulier, cette substance est un lyophilisat.

Dans le cas où le réservoir comporte deux compartiments, le poussoir peut comporter une section tubulaire latérale sensiblement cylindrique ménagée entre les parois latérales extérieures du réservoir et les parois latérales intérieures du capuchon et la longueur de cette section tubulaire latérale correspond à la course du réservoir requise pour amener l'organe d'obturation intermédiaire en appui contre l'organe obturateur et pour faire pénétrer partiellement l'organe obturateur dans le deuxième compartiment.

Dans ce cas également, la section tubulaire latérale du poussoir peut avoir un diamètre intérieur sensiblement égal au diamètre extérieur de la partie réservoir correspondant au deuxième compartiment.

La présente invention sera mieux comprise en référence à la description d'un exemple de réalisation et du dessin annexé dans lequel :

la fig. 1 représente une vue en coupe axiale d'une première forme de réalisation d'une seringue d'injection en position de stockage,

la fig. 2 représente cette même seringue en position activée,

la fig. 3 représente une vue en coupe axiale d'un deuxième mode de réalisation d'une seringue représentée en position de stockage,

la fig. 4 représente cette même seringue en position activée,

la fig. 5 illustre une vue agrandie d'un embout de pulvérisation nasale d'un appareil selon l'invention,

la fig. 6 représente une vue agrandie d'un embout du type compte-gouttes adapté à l'extrémité d'un appareil selon l'invention,

la fig. 7 illustre un mode de réalisation d'une seringue multidose correspondant à l'appareil selon l'invention dans sa position de stockage,

la fig. 8 illustre cette même seringue dans sa position activée,

la fig. 9 représente une vue agrandie d'une partie de la seringue représentée par les figures 7 et 8,

la fig. 10 représente une vue partielle d'une variante de réalisation de la seringue des fig. 3 et 4, en position de stockage,

la fig. 11 illustre la seringue de la fig. 10 dans la phase de transfert du liquide du premier compartiment vers le deuxième compartiment, et

la fig. 12 représente une vue partielle de la seringue des fig. 10 et 11 dans une phase précédant immédiatement son utilisation.

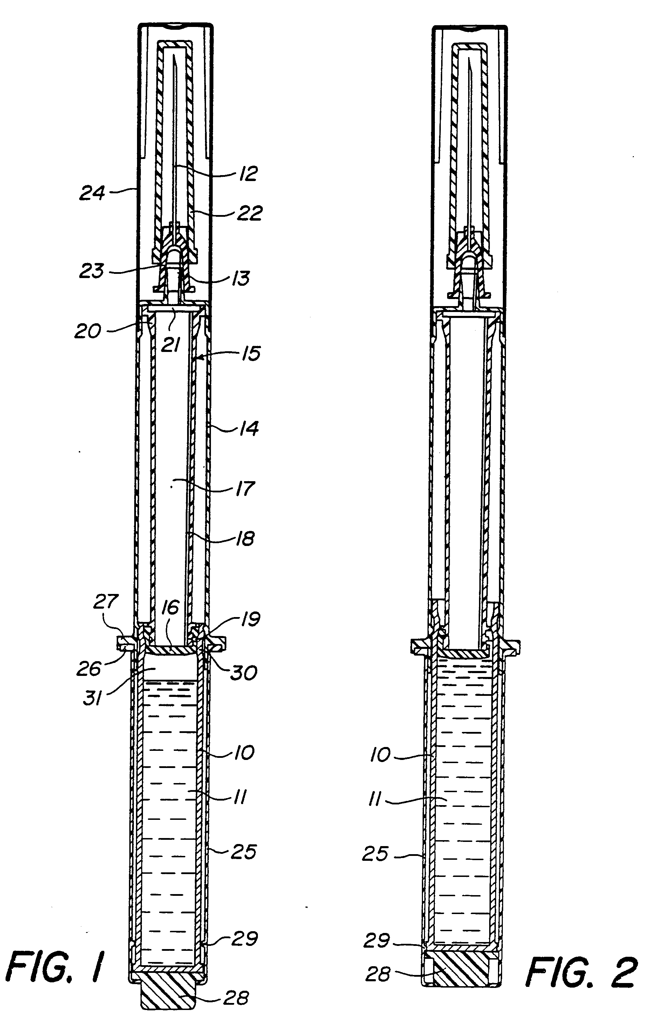

En référence aux figures 1 et 2, la seringue représentée respectivement à l'état stocké et à l'état activé comporte essentiellement un réservoir 10 contenant un liquide médicamenteux 11 à injecter au moyen d'une aiguille 12 fixée sur un embout porte-aiguille 13 solidaire d'une capsule 14. La capsule 14 porte un organe distributeur 15 qui est équipé d'un organe obturateur conçu pour jouer successivement le rôle d'obturateur proprement dit et de piston-vanne au cours d'une phase ultérieure. l'organe obturateur 16 est monté à une des extrémités d'une tige centrale 17 pourvue d'un canal axial 18 qui communique avec un canal radial 19. La tige 17 est fixée au moyen d'une embase 20 à l'une des extrémités de la capsule 14 de manière à emprisonner un filtre à membrane 21 sur lequel débouche le canal axial 18.

L'aiguille 12 est protégée par un cache 22 directement engagé sur un support d'aiguille 23. Un deuxième cache 24 dont le diamètre est sensiblement égal au diamètre de la capsule 14, est monté à l'extrémité de cette dernière pour recouvrir l'aiguille 12 et son cache 22.

Un capuchon 25 recouvre entièrement le réservoir 10. A l'une de ses extrémités, le capuchon 25 comporte des pattes 26 qui sont engagées dans un évidemment annulaire des ailettes 27 solidaires de la capsule 14. Une liaison par soudure aux ultrasons des pattes 26 avec les ailettes 27 permet d'assurer l'inviolabilité du système. A son autre extrémité, le capuchon 25 est ouvert et équipé d'un poussoir 28 dont la base est en appui sur le fond du réservoir 10. Une ou plusieurs butées internes 29 équipent le capuchon 25 et ont pour rôle de limiter la course du poussoir 28.

Dans la position de stockage, l'organe obturateur 16 est partiellement engagé à l'intérieur du col rétréci 30 du réservoir 10. De cette manière, le canal radial 19 est obturé et quelle que soit la position de la seringue, le liquide médicamenteux 11 ne peut pas s'écouler en direction de l'aiguille 12. Par ailleurs, un certain volume d'air 31, en légère surpression, surmonte le liquide 11. Pour amener la seringue dans son état activé, il convient de pousser l'organe obturateur 16 à l'intérieur du réservoir 10 de telle manière que l'embouchure du canal radial 19 débouche dans la partie large de ce réservoir, c'est-à-dire au-delà du col à section rétrécie 30. Pour ce faire, il est nécessaire de vaincre les forces dues à la résistance élastique à la compression de l'organe obturateur 16, réalisé en un matériau élastomère, et de comprimer le volume d'air 31 contenu dans le réservoir 10. Comme mentionné en préambule, la force appliquée pour vaincre ces résistances est relativement importante et doit être appliquée sur le fond du réservoir 10 de manière à engendrer un déplacement relatif de ce réservoir et de l'organe obturateur 16. Par ailleurs, il est nécessaire que ces forces soient appliquées d'une manière contrôlée pour éviter que l'organe obturateur pénètre trop profondément dans le réservoir et qu'une partie du liquide 11 ne s'échappe accidentellement par le canal radial 19, le canal axial 18 et l'aiguille 12. Pour éviter ce risque, le capuchon 25 est pourvu du poussoir 28 et d'au moins d'une butée d'arrêt 29 qui limite la course de ce poussoir 28. Cette course est déterminée de telle manière qu'elle corresponde au déplacement souhaité du réservoir 10 par rapport à l'organe obturateur 16, ce déplacement étant suffisant pour "débuller" la seringue et éventuellement même faire apparaître la première goutte de liquide injectable à l'extrémité de l'aiguille.

Lorsque cette opération est terminée, le capuchon 25 peut être retiré ainsi que les caches 12 et 24 et l'opérateur peut effectuer l'injection.

La figure 2 représente la seringue en position activée après enfoncement du poussoir 28 jusqu'à la butée d'arrêt 29. Cette figure montre clairement que le volume d'air 31 a été évacué et que la seringue est pratiquement prête à l'injection, à condition toutefois que le capuchon 25 et les caches 22 et 24 soient retirés.

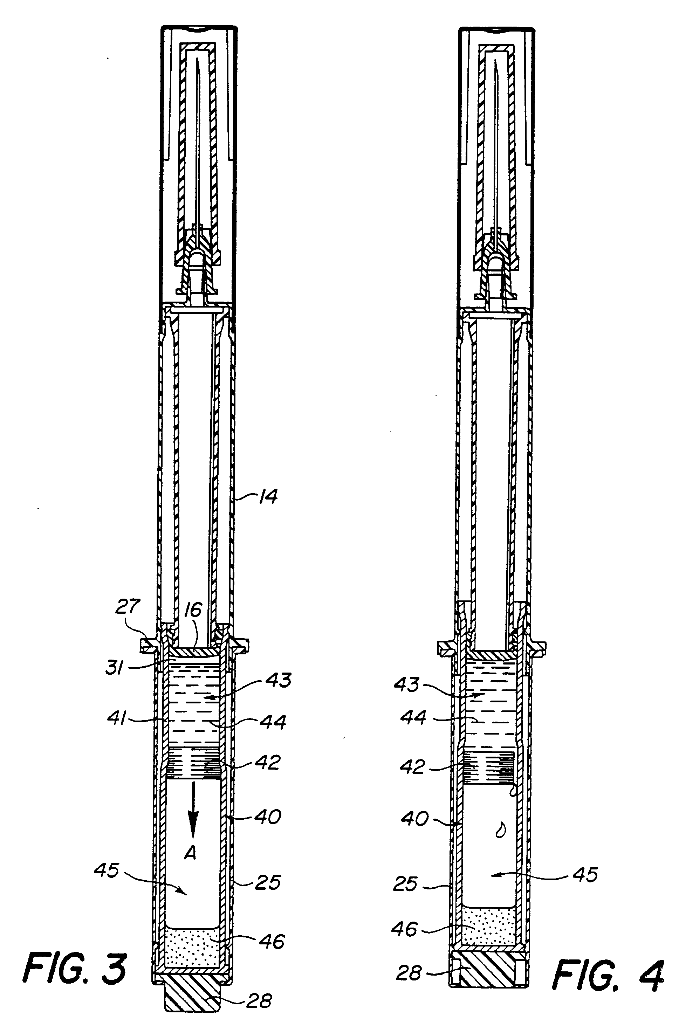

La seringue illustrée par les figures 3 et 4, respectivement en position de stockage et en position activée, diffère de celle des figures 1 et 2 en ce que le réservoir 40 est du type à double compartiment. Ce réservoir comprend un col rétréci 41 relativement long qui est obturé à l'extrémité ouverte dudit réservoir par un organe obturateur 16 en élastomère identique à celui de la seringue des figures 1 et 2, et à son autre extrémité par un deuxième organe obturateur 42, qui sera appelé par la suite organe d'obturation intermédiaire, également en élastomère. Le premier compartiment 43, délimité d'une part par la paroi du col à section rétrécie 41 et d'autre part par l'organe obturateur 16 et l'organe d'obturation intermédiaire 42, contient un liquide 44 qui est destiné à être mélangé à une substance contenue dans le deuxième compartiment 45 délimité d'une part par la paroi intérieure élargie du réservoir 40 et d'autre part par l'organe d'obturation intermédiaire 42 et le fond dudit réservoir 40. Dans le présent cas la substance contenue dans le deuxième compartiment 45 est un lyophilisat 46 et le liquide 44 contenu dans le premier réservoir est un solvant destiné à dissoudre ce lyophilisat.

Dans sa position de stockage, l'organe d'obturation intermédiaire sépare de façon étanche les deux compartiments 43 et 45. Dans une première phase d'utilisation, l'opérateur doit pouvoir repousser l'organe d'obturation intermédiaire 42 hors du col rétréci de telle manière que le solvant 44 soit libéré et puisse s'écouler dans le deuxième compartiment pour dissoudre le lyophilisat 46. Dans ce but, l'opérateur enfonce le poussoir 28 ce qui a pour effet de déplacer le réservoir 40 par rapport à l'organe obturateur 16 qui pénètre plus en avant à l'intérieur du col de section rétrécie 41. Après une première phase de compression du volume de gaz 31 surmontant le liquide 44, la pression exercée sur le contenu du premier compartiment 43 par l'organe obturateur 16 est intégralement transmise à l'organe d'obturation intermédiaire 42 qui se déplace dans le sens de la flèche A. La course du poussoir 28 est déterminée de telle manière que le déplacement de l'organe d'obturation intermédiaire 42 soit suffisant pour libérer le solvant 44. Le mélange ou la dissolution du lyophilisat peut alors se faire et la seringue sera prête à l'injection après le retrait du capuchon 25, suite à la rupture de ses liens d'inviolabilité avec les ailettes 27 de la capsule 14, et l'opération de "débullage" qui consiste à déplacer le réservoir de manière à amener l'organe d'obturation intermédiaire 42 en contact avec l'organe obturateur 16 et à faire pénétrer ce dernier dans le deuxième compartiment.

Dans cet exemple de réalisation, le poussoir 28 permet d'assurer un déplacement limité mais suffisant pour activer la seringue tout en évitant l'éjection accidentelle d'une partie du solvant 44 avant qu'il ait eu le temps de s'écouler dans le deuxième compartiment pour dissoudre le lyophilisat.

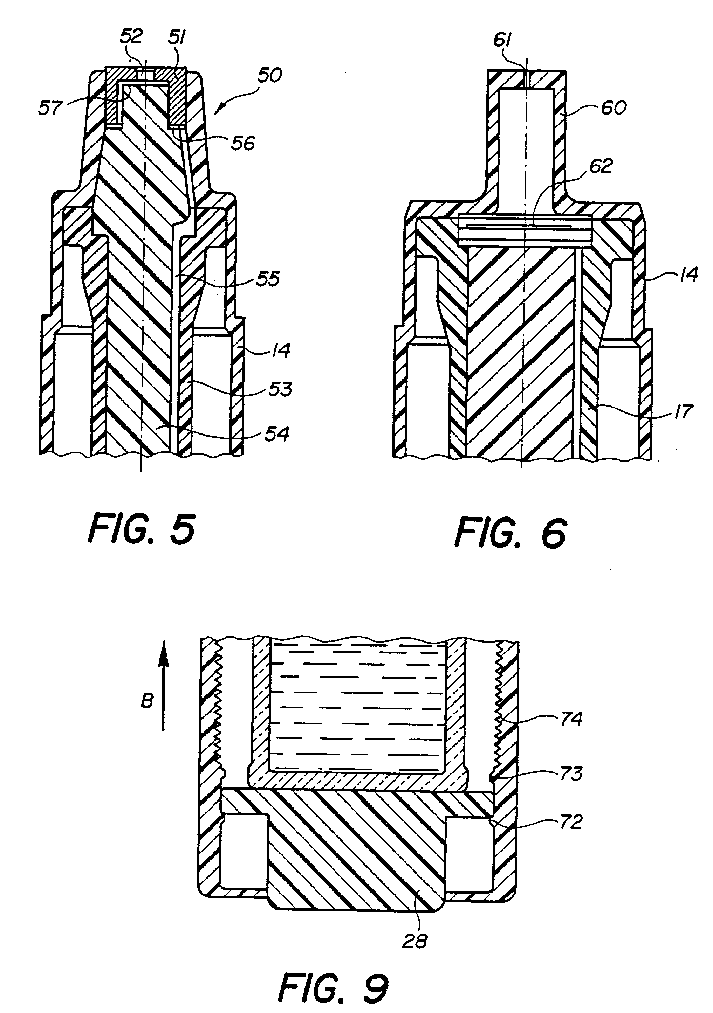

La figure 5 illustre une vue en coupe partielle d'une autre forme de réalisation d'un appareil distributeur qui est équipé d'un embout 50 de pulvérisation nasale. Dans cet exemple de réalisation, la capsule 14 porte à son extrémité une pièce 51 pourvue d'une ouverture 52 constituant une buse de pulvérisation. Le support 53 de l'organe d'obturation (non représenté) a une forme tubulaire et contient un noyau plein 54 conçu de manière à définir un canal axial 55 qui débouche sur un évidement annulaire 56 ménagé à l'extrémité intérieure de la pièce 51, et qui communique avec un canal 57 débouchant sur la buse de pulvérisation 52. Le fonctionnement de l'appareil est sensiblement identique à celui décrit en référence aux figures 1 et 2. Le fait d'enfoncer le poussoir (non représenté) provoque l'activation de l'appareil, c'est-à-dire la pénétration de l'organe obturateur à l'intérieur du réservoir correspondant.

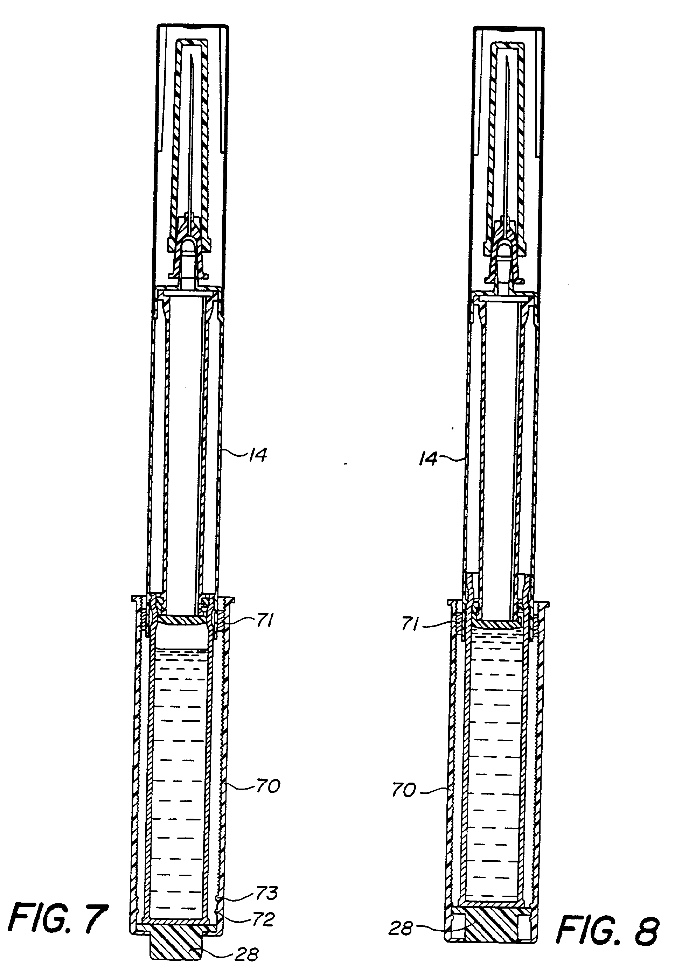

La figure 6 illustre un autre mode de réalisation de l'appareil distributeur qui est dans ce cas équipé d'un embout du type compte-gouttes. Dans ce cas l'extrémité de la capsule 14 comporte un embout rétréci 60 pourvu d'une ouverture centrale 61. Un filtre du type à membrane 62 peut être interposé entre l'embout 60 et l'embase du support 17 de l'organe obturateur (non représenté).

Les figures 7 et 8 illustrent un appareil distributeur, qui dans ce cas, est une seringue d'injection, du type multidose. Ce qui caractérise cet appareil par rapport aux seringues représentées par les figures 1 et 2 est l'existence d'un capuchon 70 fileté intérieurement qui est vissé sur une pièce complémentaire 71 filetée extérieurement et fixée à l'extrémité supérieure de la capsule 14, le but recherché par cette réalisation consistant à permettre le déplacement relatif contrôlé du capuchon 70, par rapport à la capsule 14.

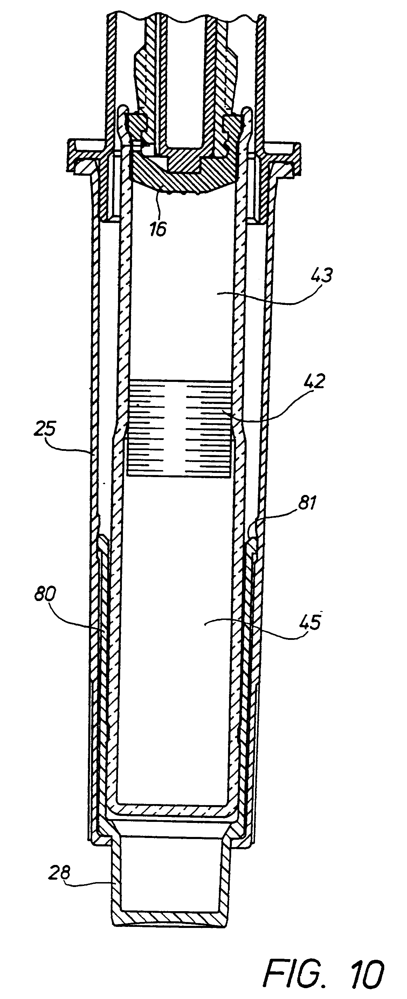

Dans la position de stockage représentée par la figure 7, le poussoir 28 est dans sa position extrême. Pour activer le système il convient d'enfoncer ce poussoir au-delà d'une première butée 72 jusqu'à une deuxième butée 73. Cette opération est illustrée par la figure 8. Le poussoir est alors entièrement enfoncé à l'intérieur du capuchon 70 et la seringue est activée, c'est-à-dire "débullée" et prête à l'utilisation. Dans ce cas, l'opérateur ne retire pas le capuchon 70 contrairement à ce qu'il effectue avec le capuchon 25 de la seringue des figures 1 et 2, mais il visse ce capuchon sur la pièce filetée 71, ce qui a pour effet de le déplacer dans le sens de la flèche B comme le montre la figure 9. Ce déplacement relatif refoule le poussoir 28 en appui contre la première butée 72. Dans cette position, la seringue est prête pour l'injection d'une première dose. Cette première dose correspond au déplacement du poussoir entre la première butée 72 et deuxième butée 73. Après cette première injection, l'opérateur visse à nouveau le capuchon 70 sur l'élément fileté 71 pour ramener le poussoir 28 dans sa position haute, ce qui a pour effet de réactiver le système et de le rendre prêt à l'injection de la dose suivante.

Il est bien entendu que ce principe ne s'applique pas uniquement aux appareils distributeurs du type seringue, mais également à toute autre forme d'appareils distributeurs tels que les pulvérisateurs, les distributeurs de gouttes, etc.

Par ailleurs, le filetage intérieur 74 peut être remplacé par une structure équivalente au point de vue du fonctionnement telle que par exemple une rainure hélicoïdale ou similaire.

En référence aux figures 10 à 12, la seringue du type mélangeuse ou à deux compartiments 43 et 45 est identique à celle des figures 3 et 4, sauf en ce qui concerne la forme et la fonction du poussoir 28. Comme précédemment, elle comporte un organe obturateur 16 qui joue le rôle d'un obturateur, d'un piston et d'une vanne, et un organe d'obturation intermédiaire 42 qui sépare le premier compartiment 43 du deuxième compartiment 45.

Le capuchon 25 comporte une ouverture dans laquelle est engagé le poussoir 28. Ce dernier comporte une section sensiblement cylindrique 80 qui est engagée entre les parois latérales extérieures du réservoir et les parois latérales intérieures du capuchon. La longueur axiale de cette section est calculée pour que ce poussoir puisse assurer ses nouvelles fonctions qui ressortiront de la description ci-dessous.

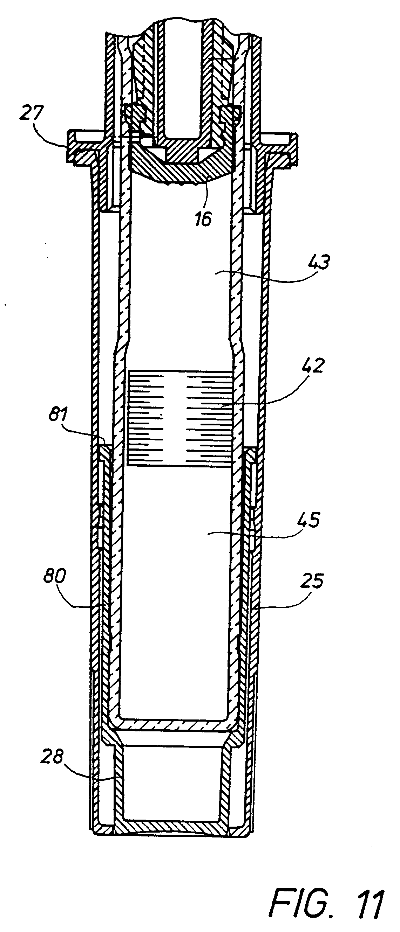

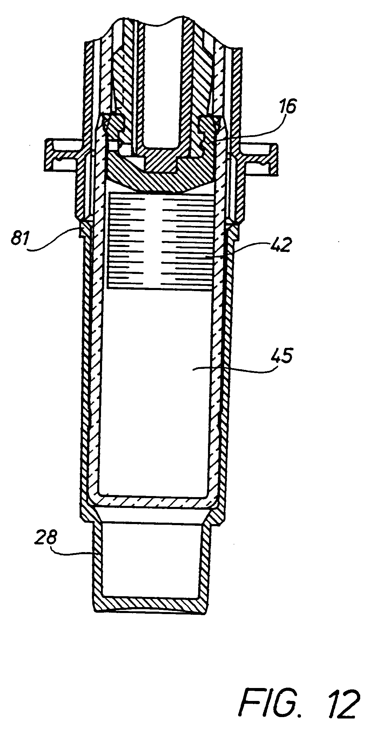

Dans la phase de stockage, poussoir 28 et capuchon 25 occupent la position relative illustrée par la figure 10. Dans un premier temps, l'opérateur enfonce le fond du poussoir 28 dans le capuchon dont la base est en appui contre les ailettes 27 de l'appareil. Ceci a pour effet de faire pénétrer l'organe obturateur 16 dans le col rétréci du premier compartiment 43 et de provoquer la pénétration de l'organe d'obturation intermédiaire 42 dans le second compartiment. le liquide du premier compartiment peut ainsi s'écouler dans le second compartiment. Cette phase est illustrée par la figure 11. A ce moment, l'opérateur retire le capuchon 25 et enfonce le poussoir jusqu'à ce que le bord libre 81 de la section cylindrique 80 vienne en butée contre le rebord de la capsule (fig. 12). Tout le liquide du premier compartiment 43 a été intégralement transféré dans le deuxième compartiment 45. L'organe d'obturation intermédiaire 42 est amené en contact avec l'organe obturateur 16. Ce dernier a pénétré partiellement dans le deuxième compartiment, c'est-à-dire dans la section élargie du réservoir. Après retrait du poussoir 28, l'injection peut avoir lieu.

Ce dispositif est particulièrement intéressant pour l'utilisateur parce qu'il permet une "préparation" quasi automatique de la seringue par des manipulations élémentaires.

## Revendications

1. Appareil distributeur de substances liquides, en particulier de produits médicamenteux, comportant un réservoir (10) allongé ouvert à une de ses extrémités, comprenant un fond fermé à son extrémité opposée, des parois latérales sensiblement cylindriques et un col rétréci (30), et contenant au moins un liquide à mélanger à une autre substance ou à distribuer, par exemple par pulvérisation, par application cutanée ou oculaire, ou par injection; et un organe distributeur, mobile par rapport à ce réservoir, cet organe distributeur comportant au moins un organe obturateur (16), cet organe obturateur, comportant un canal axial (18) raccordé à un canal radial (19) et étant partiellement engagé dans le col rétréci du réservoir (10) pour obturer ce réservoir lorsque l'appareil est dans une première position de stockage et pour libérer le liquide lorsque l'appareil est dans une deuxième position activée, ledit canal radial (19) étant obturé dans ladite première position, son embouchure se trouvant en appui contre les parois du col rétréci, et ce canal radial (19) étant dégagé dans ladite deuxième position, son embouchure étant libre du fait que l'organe obturateur est refoulé vers l'intérieur du réservoir (10); ainsi qu'une capsule (14) solidaire de cet organe obturateur (16) et montée sur le réservoir (10) de telle manière qu'un tronçon d'extrémité de ce réservoir, voisin de son col rétréci, soit engagé dans cette capsule; ce réservoir étant coiffé d'un capuchon (25) équipé d'organes d'inviolabilité le rendant solidaire de la capsule (14) pendant le stockage; caractérisé en ce que ledit capuchon (25) est pourvu d'un poussoir (28) qui est agencé pour prendre appui sur au moins une partie du fond fermé du réservoir et/ou de ses parois latérales, et qui est conçu pour pousser le réservoir (10) de telle manière que l'organe obturateur (16) pénètre dans le col (30) et que l'appareil passe de sa position de stockage à sa position activée, ce poussoir (28) étant une pièce mobile axialement par rapport audit capuchon (25).

2. Appareil selon la revendication 1, caractérisé en ce que l'organe distributeur est une aiguille (12) de seringue et que le liquide à distribuer est un médicament injectable.

3. Appareil selon la revendication 1, caractérisé en ce que l'organe distributeur est un applicateur cutané, oculaire ou un pulvérisateur et en ce que le liquide est une substance à distribuer par voie cutanée, oculaire ou par pulvérisation.

4. Appareil selon la revendication 1, caractérisé en ce que le capuchon comporte au moins une butée d'arrêt pour limiter la course du poussoir.

5. Appareil selon la revendication 1, dans lequel le réservoir contient plusieurs doses de liquide à distribuer, caractérisé en ce que le capuchon comporte deux butées d'arrêt (72, 73) agencées pour définir la course du poussoir pour chaque dose à distribuer, et des moyens pour assurer un déplacement axial contrôlé du capuchon pour ramener le poussoir dans sa position initiale après chaque distribution d'une dose de liquide.

6. Appareil selon la revendication 5, caractérisé en ce que les moyens pour assurer un déplacement axial contrôlé du capuchon comprennent un filetage intérieur (74) de ce capuchon et un élément complémentaire agencé pour s'engager dans ce filetage et qui est solidaire de la capsule.

7. Appareil selon la revendication 1, dans lequel le réservoir comporte une dose unitaire d'un seul liquide à distribuer, caractérisé en ce que la course du poussoir est égale au déplacement axial du réservoir, par rapport à l'organe d'obturation, nécessaire pour amener la première goutte de liquide à une extrémité de distribution de l'organe distributeur.

8. Appareil selon la revendication 1, dans lequel le réservoir comporte deux compartiments (43, 45) séparés par un organe d'obturation intermédaire (42) et dans lequel le compartiment disposé entre l'organe obturateur (16) et l'organe d'obturation intermédiaire (42), et correspondant au col rétréci du réservoir, contient un liquide destiné à être mélangé à une substance contenue dans le deuxième compartiment disposé entre l'organe d'obturation intermédiaire et le fond du réservoir, caractérisé en ce que la course du poussoir est égale du déplacement axial du réservoir, par rapport à l'organe obturateur, nécessaire pour assurer l'éjection de l'organe d'obturation intermédiaire hors du col rétréci et le passage du liquide du premier compartiment dans le deuxième compartiment.

9. Appareil selon la revendication 8, caractérisé en ce que le deuxième compartiment (45) contient une substance à mélanger au liquide du premier compartiment.

10. Appareil selon la revendication 9, caractérisé en ce que le deuxième compartiment (45) contient un lyophilisat.

11. Appareil selon la revendication 8, caractérisé en ce que ledit poussoir comporte une section tubulaire (80) latérale sensiblement cylindrique ménagée entre les parois latérales extérieures du réservoir et les parois latérales intérieures du capuchon et en ce que la longueur de cette section tubulaire latérale correspond à la course du réservoir requise pour amener l'organe d'obturation intermédiaire en appui contre l'organe obturateur et pour faire pénétrer partiellement l'organe obturateur dans le deuxième compartiment.

12. Appareil selon la revendication 11, caractérisé en ce que la section tubulaire (80) latérale du poussoir a un diamètre intérieur sensiblement égal au diamètre extérieur de la partie réservoir correspondant au deuxième compartiment.

## Patentansprüche

1. Vorrichtung für das Verteilen von flüssigen Stoffen, insbesondere von medikamentösen Produkten, bestehend aus einem langgezogenen Behälter (10), der an einem seiner Enden offen ist, einen geschloßenen Boden an dem entgegengesetzten Ende, im wesentlichen zylindrische seitliche Wandungen und einen eingeschnürten Hals (30) aufweist und mindestens eine Flüssigkeit enthält, die mit einem anderen Stoff zu vermischen oder zu verteilen ist, beispielsweise durch Versprühen, durch Verabreichen auf die Haut oder in die Augen oder durch Injektion, und aus einem Verteilorgan, das gegenüber diesem Behälter beweglich ist, wobei dieses Verteilorgan mindestens einen Verschlußteil (16) enthält, wobei dieser Verschlußteil eine axiale Leitung (18) enthält, die mit einer radialen Leitung (19) verbunden ist und teilweise in den eingeschnürten Hals des Behälters (10) eingeführt ist, um diesen Behälter zu verschließen, wenn die Vorrichtung sich in einer ersten Lagerstellung befindet, und die Flüssigkeit abfließen zu lassen, wenn die Vorrichtung sich in einer zweiten Einsatzstellung befindet, wobei die besagte radiale Leitung (19) in der besagten ersten Stellung verschlossen ist, da deren Ansatz gegen die Wandungen des eingeschnürten Halses anliegt und diese radiale Leitung (19) in der besagten zweiten Stellung frei ist, indem ihr Ansatz frei ist, da der Verschlußteil in das Innere des Behälters (10) gedrängt ist, sowie aus einer Kapsel (14), die mit diesem Verschlußteil (16) fest verbunden und auf dem Behälter (10) derart aufgesetzt ist, daß ein Teil des Endes dieses Behälters (10) in der Nähe seines eingeschnürten Halses in diese Kapsel (14) eingeführt ist, wobei dieser Behälter (10) eine Kappe (25) trägt, die mit Teilen für eine Unverletzbarkeit versehen ist, wodurch während der Lagerung der Behälter (10) mit der Kapsel (14) fest verbunden ist, dadurch gekennzeichnet, daß die besagte Kappe (25) mit einem Drücker (28) versehen ist, der eingerichtet ist, um auf mindestens einem Teil des geschlossenen Bodens des Behälters (10) und/oder dessen seitlicher Wandungen abgestützt zu werden, und der ausgelegt ist, um den Behälter (10) derart zu schieben, daß der Verschlußteil (16) in den Hals (30) eindringt und daß die Vorrichtung von ihrer Lagerstellung in ihre Einsatzstellung übergeht, wobei dieser Drücker (28) ein Teil ist, der gegenüber der besagten Kappe (25) axial beweglich ist.

2. Vorrichtung gemäß Anspruch 1, dadurch gekennzeichnet, daß das Verteilorgan eine Injektionsnadel (12) und die Flüssigkeit, die zu verteilen ist, ein injizierbares Arzneimittel ist.

3. Vorrichtung gemäß Anspruch 1, dadurch gekennzeichnet, daß das Verteilorgan für ein Verabreichen auf die Haut, in die Augen oder durch Versprühen ausgebildet und die Flüssigkeit ein Stoff ist, der durch ein Verabreichen auf die Haut, in die Augen oder durch Versprühen zu verteilen ist.

4. Vorrichtung gemäß Anspruch 1, dadurch gekennzeichnet, daß die Kappe mit mindestens einem Anschlag zu versehen ist, um den Verstellweg des Drückers zu begrenzen.

5. Vorrichtung gemäß Anspruch 1, bei der der Behälter mehrere Dosen der Flüssigkeit, die zu verteilen ist, enthält, dadurch gekennzeichnet, daß die Kappe zwei Anschläge (72,73) aufweist, die eingerichtet sind, um den Verstellweg des Drückers für jede Dosis, die zu verteilen ist, festzulegen und Einrichtungen, um eine kontrollierte axiale Verstellung der Kappe sicherzustellen, damit nach jeder Verteilung einer Dosis der Flüssigkeit der Drücker in seine ursprüngliche Stellung zurückgebracht wird.

6. Vorrichtung gemäß Anspruch 5, dadurch gekennzeichnet, daß die Einrichtungen für die Sicherstellung einer kontrollierten axialen Verstellung der Kappe aus einem Innengewinde (74) in dieser Kappe und aus einem Zusatzteil, der eingerichtet ist, um in dieses Gewinde eingeführt zu werden, und mit der Kapsel fest verbunden ist, bestehen.

7. Vorrichtung gemäß Anspruch 1, bei der der Behälter eine Einzeldosis von einer einzigen Flüssigkeit aufweist, die zu verteilen ist, dadurch gekennzeichnet, daß der Verstellweg des Drückers mit der axialen Verstellung des Behälters gegenüber dem Verschlußteil identisch ist, die erforderlich ist, um den ersten Tropfen der Flüssigkeit an ein Ende für die Verteilung an dem Verteilorgan zu bringen.

8. Vorrichtung gemäß Anspruch 1, bei der der Behälter zwei Abteile (43,45) aufweist, die von einem Zwischenverschlußteil (42) getrennt sind, und bei der das Abteil, das zwischen dem ersten Verschlußteil (16) und dem Zwischenverschlußteil (42) angeordnet ist und dem eingeschnürten Hals des Behälters entspricht, eine Flüssigkeit enthält, die dafür bestimmt ist, mit einem Stoff, der in dem zweiten Abteil enthalten ist, vermischt zu werden, wobei das zweite Abteil zwischen dem Zwischenverschlußteil und dem Behälterboden abgegrenzt ist, dadurch gekennzeichnet, daß der Verstellweg des Drückers mit der axialen Verstellung des Behälters gegenüber dem Verschlußteil identisch ist, die erforderlich ist, um das Abstoßen des Zwischenverschlußteils aus dem eingeschnürten Hals und das Abfließen der Flüssigkeit aus dem ersten Abteil in das zweite Abteil sicherzustellen.

9. Vorrichtung gemäß Anspruch 8, dadurch gekennzeichnet, daß das zweite Abteil (45) einen Stoff enthält, der mit der Flüssigkeit in dem ersten Abteil zu vermischen ist.

10. Vorrichtung gemäß Anspruch 9, dadurch gekennzeichnet, daß das zweite Abteil (45) ein Lyophilisat enthält.

11. Vorrichtung gemäß Anspruch 8, dadurch gekennzeichnet, daß der besagte Drücker einen seitlichen, rohrförmigen, im wesentlichen zylindrischen Abschnitt aufweist, der zwischen den seitlichen außenliegenden Wandungen des Behälters und den seitlichen innenliegenden Wandungen der Kappe eingerichtet ist, und daß die Länge dieses seitlichen, rohrförmigen Abschnitts dem erforderlichen Verstellweg des Behälters entspricht, damit der Zwischenverschlußteil gegen den Verschlußteil anliegt und der Verschlußteil in das zweite Abteil teilweise eingeführt werden kann.

12. Vorrichtung gemäß Anspruch 11, dadurch gekennzeichnet, daß der seitliche, rohrförmige Abschnitt (80) des Drückers einen inneren Durchmesser aufweist, der im wesentlichen mit dem äußeren Durchmesser des Teils des Behälters, der dem zweiten Abteil entspricht, identisch ist.

**Claims**

1. Device for the distribution of liquid substances, especially of medical products, comprising an elongated reservoir (10) open at one end, and a base closed at its opposite end, substantially cylindrical side walls and a narrowed neck (30), and containing at least one liquid to be mixed with another substance or to be distributed, for example by spraying, by application to the skin or eyes, or by injection; and a distribution means mobile with respect to this reservoir, this distribution means comprising at least one stopper means (16), this stopper means, comprising an axial canal (18) connected with a radial canal (19) and being partially engaged in the narrowed neck of the reservoir (10) to close this reservoir when the device is in a first storage position and to liberate the liquid when the device is in a second activated position said radial canal (19) being closed in said first position, its opening pressing against the walls of the narrowed neck, and this radial canal (19) being free in said second position, its opening being free because the stopper means is driven back inside the reservoir (10); also a capsule (14) solid with this stopper means (16) and mounted on the reservoir (10) so that a protruding section of this reservoir, neighbouring its narrowed neck, is engaged in this capsule; this reservoir being surmounted by a barrel (25) provided with inviolability units keeping it firmly attached to the capsule (14) during storage; characterized in that said barrel (25) is provided with a plunger (28) designed to push against at least one part of the closed base of the reservoir and/or against its side walls, and which is designed to push the reservoir (10) in such a way that the stopper means (16) enters the neck (30) and the device passes from its storage position to its activated position, this plunger (28) being a piece moving axially with respect to said barrel (25).

2. Device according to claim 1, characterized in that the distribution means is a syringe needle (12) and that the liquid to be distributed is an injectable medicine.

3. Device according to claim 1, characterized in that the distribution means is an applicator for the skin, the eyes or of a spray and in that the liquid is a substance to be applied to the skin or the eyes or by spraying.

4. Device according to claim 1, characterized in that the barrel comprises at least one stop to limit the course of the plunger.

5. Device according to claim 1, in which the reservoir contains several doses of liquid to be distributed, characterized in that the barrel carries two stops (72, 73) designed to define the path of the plunger for each dose to be distributed, and means of ensuring a controlled axial movement of the barrel to bring the plunger back into its initial position after each distribution of a dose of liquid.

6. Device according to claim 5, characterized in that the means of ensuring a controlled axial movement of the barrel comprise an interior thread (74) of its barrel and a complementary element designed to engage in this thread and which is solid with the capsule.

7. Device according to claim 1, in which the reservoir contains a single dose of a single liquid to be distributed, characterized in that the path of the plunger equals the axial displacement of the reservoir, with respect to the stopper means, necessary to bring the first drop of liquid to an extremity of distribution of the distribution means.

8. Device according to claim 1, in which the reservoir comprises two compartments (43, 45) separated by an intermediate stopper means (42) and in which the compartment positioned between the stopper means (16) and the intermediate stopper means (42), and corresponding to the narrowed neck of the reservoir, contains a liquid destined to be mixed with a substance contained in the second compartment situated between the intermediate stopper means and the base of the reservoir, characterized in that the path of the plunger is equal to the axial displacement of the reservoir, with respect to the stopper means, necessary to ensure the ejection of the intermediate stopper means by the narrowed neck and the passage of the liquid from the first compartment into the second compartment.

9. Device according to claim 8, characterized in that the second compartment (45) contains a substance to be mixed with the liquid in the first compartment.

10. Device according to claim 9, characterized in that the second compartment (45) contains a lyophilisate.

11. Device according to claim 8, characterized in that said plunger comprises an appreciably cylindrical tubular lateral section (80) positioned between the exterior side walls of the reservoir and the interior side walls of the barrel and in that the length of each tubular side section corresponds to the path of the reservoir required to make the intermediary stopper means press against the stopper means and to make the stopper means partially penetrate the second compartment.

12. Device according to claim 11, characterized in that the lateral tubular section (80) of the plunger has an interior diameter substantially equal to the exterior diameter of the part of the reservoir corresponding to the second compartment.

FIG. 1

FIG. 2

FIG. 3

FIG. 4

**FIG. 5**

**FIG. 6**

**FIG. 9**

FIG. 7

FIG. 8

FIG. 10

FIG. 11

FIG. 12